# NOUVEAU FASCICULE DE BREVET EUROPEEN

(11) **EP 1 096 916 B2**
(45) Date de publication et mention de la décision concernant l'opposition: **19.03.2014**
(45) Mention de la délivrance du brevet: 19.08.2009
(21) Numéro de dépôt: 99923706.8
(22) Date de dépôt: 09.06.1999
(51) Int. Cl.: A61K 8/35, A61K 8/81, A61Q 17/04

(54) **PROCEDE DE PHOTOSTABILISATION DE DERIVES DU DIBENZOYLMETHANE**
VERFAHREN ZUR PHOTOSTABILISIERUNG VON DIBENZOYLMETHANDERIVATEN
METHOD FOR PROVIDING DIBENZOYLMETHANE DERIVATIVES WITH LIGHT STABILITY

(30) Priorité: 08.07.1998 FR 9808765
(43) Date de publication de la demande: 09.05.2001
(73) Titulaire: Air Products and Chemicals, Inc., Allentown, PA 18195-1501 (US)
(72) Inventeur: HANSENNE, Isabelle, Westfield, NJ 07090 (US); JOSSO, Martin, F-75005 Paris (FR)
(74) Mandataire: Andrae Westendorp
(86) Numéro de dépôt international: PCT/FR1999/001366
(87) Numéro de publication internationale: WO 2000/002528

(56) Documents cités:
- EP-A- 0 685 227
- EP-A2- 0 845 260
- WO-A1-93/01797
- WO-A1-94/04131
- WO-A1-97/28785
- FR-A- 2 398 496
- US-A- 5 736 125
- US-A- 5 736 125
- Fiche INCI pour "Acrylates/Viny/Isodecanoate Crosspolymer"
- Fiche technique pur "Stabylen 30"
- Fiche technique pour "Pemulen"

## Description

La présente invention concerne, entre autres objets, de nouvelles compositions cosmétiques à usage topique spécifiquement destinées à la photoprotection de la peau et/ou des cheveux contre le rayonnement ultraviolet (compositions ci-après dénommées plus simplement compositions antisolaires ou compositions filtrantes), leur utilisation dans l'application cosmétique susmentionnée, ainsi qu'un procédé général de photostabilisation de filtres solaires particuliers, actifs dans l'UV-A, au moyen d'un ou plusieurs composés convenablement sélectionnés.

Plus précisément encore, elle concerne des compositions antisolaires photostables à l'égard des UV qui comprennent, dans un support cosmétiquement acceptable, au moins une phase huileuse et au moins un composé du type dérivé du dibenzoylméthane associé à au moins un copolymère épaississant particulier à titre d'agent photostabilisant, ainsi que le procédé correspondant de stabilisation dudit ou desdits filtres UV-A au moyen dudit ou desdits polymères épaississants.

On sait que les rayons UV-A, de longueurs d'onde comprises entre 320 et 400 nm, provoquent le brunissement de la peau, mais qu'ils sont également susceptibles d'induire à la longue une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent en outre le déclenchement de la réaction érythémateuse ou amplifient cette réaction chez certains sujets et peuvent même être à l'origine de réactions photo-toxiques ou photo-allergiques. Il est donc souhaitable de filtrer le rayonnement UV-A.

De nombreux filtres organiques solaires capables d'absorber plus ou moins sélectivement les rayons UV-A nocifs ont été proposés à ce jour dans le domaine de la cosmétique.

A cet égard, une famille de filtres UV-A particulièrement intéressante est actuellement constituée par les dérivés du dibenzoylméthane, et notamment le 4-(ter.-butyl) 4'-méthoxy dibenzoylméthane, qui présentent en effet un fort pouvoir d'adsorption intrinsèque. Ces dérivés du dibenzoylméthane sont maintenant des produits bien connus en soi à titre de filtres actifs dans l'UV-A lipophiles et sont notamment décrits dans les demandes de brevets français FR-A-2326405 et FR-A-2440933, ainsi que dans la demande de brevet européen EP-A-0114607 ; le 4-(ter.-butyl) 4'-méthoxy dibenzoyl-méthane est par ailleurs actuellement proposé à la vente sous la dénomination commerciale de "PARSOL 1789" par la Société GIVAUDAN.

Malheureusement, il se trouve que les dérivés du dibenzoylméthane sont des produits relativement sensibles au rayonnement ultraviolet (surtout UV-A), c'est-à-dire, plus précisément, qu'ils présentent une fâcheuse tendance à se dégrader plus ou moins rapidement sous l'action de ce dernier. Ainsi, ce manque substantiel de stabilité photochimique des dérivés du dibenzoylméthane face au rayonnement ultraviolet auquel ils sont par nature destinés à être soumis, ne permet pas de garantir une protection constante durant une exposition solaire prolongée, de sorte que des applications répétées à intervalles de temps réguliers et rapprochés doivent être effectuées par l'utilisateur pour obtenir une protection efficace de la peau contre les rayons UV.

La photostabilisation des dérivés du dibenzoylméthane vis-à-vis du rayonnement UV constitue, à ce jour, un problème qui n'a pas encore été résolu de manière complètement satisfaisante.

Or, la Demanderesse vient maintenant de découvrir, de façon inattendue et surprenante, qu'en associant aux dérivés du dibenzoylméthane mentionnés ci-dessus dans un support comprenant au moins phase huileuse une quantité efficace d'au moins un polymère épaississant particulier que l'on définira plus en détail ci-après, il est possible d'améliorer de manière substantielle et remarquable, la stabilité photochimique (ou photostabilité) de ces mêmes dérivés du dibenzoylméthane sans affecter la stabilité dans le temps de leur efficacité photoprotectrice dans l'UV-A.

Cette découverte, essentielle, est à la base de la présente invention.

Ainsi, conformément à l'un des objets de la présente invention, il est maintenant proposé un nouveau procédé de stabilisation des dérivés du dibenzoylméthane vis-à-vis du rayonnement UV (longueurs d'ondes comprises entre 280 nm et 400 nm environ), en particulier du rayonnement solaire, lorsque ces filtres sont présents dans un support contenant au moins une phase huileuse, ledit procédé étant tel que défini dans la revendication 11.

Conformément à un autre objet de la présente invention, il est également proposé de nouvelles compositions cosmétiques filtrantes photostables destinées à la protection de la peau et/ou des cheveux contre le rayonnement ultraviolet, en particulier le rayonnement solaire, telles que définies dans la revendication 15.

Par quantité efficace de copolymère épaississant conforme à l'invention, on entend une quantité suffisante pour obtenir une amélioration notable et significative de la photostabilité du ou des dérivés du dibenzoylméthane de la composition cosmétique photoprotectrice. Cette quantité minimale en agent stabilisant à mettre en oeuvre, qui peut varier selon la nature du support cosmétiquement acceptable retenu pour la composition, peut être déterminée sans aucune difficulté au moyen d'un test classique de mesure de photostabilité, tel que celui donné dans les exemples ci-après.

La présente invention a également enfin pour objet l'utilisation polymères épaississants spécifiques que l'on définira plus en détail ci-après pour stabiliser vis-à-vis des rayons UV un dérivé du dibenzoylméthane contenu dans une composition cosmétique comprenant au moins une phase huileuse.

D'autres caractéristiques, aspects et avantages de la présente invention apparaîtront à la lecture de la description détaillée qui va suivre.

Comme indiqué précédemment, les dérivés du dibenzoylméthane destinés à être photostabilisés dans le cadre de la présente invention sont des produits déjà bien connus en soi et décrits notamment dans les documents FR-A-2326405, FR-A-2440933 et EP-A-0 114 607 précités, documents dont les enseignements sont, pour ce qui touche à la définition même de ces produits, totalement inclus à titre de références dans la présente description.

Selon la présente invention, on peut bien entendu mettre en oeuvre un ou plusieurs dérivés du dibenzoylméthane.

Parmi les dérivés du dibenzoylméthane rentrant particulièrement bien dans le cadre de la présente invention, on peut notamment citer, de manière non limitative :
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4,4'-diméthoxydibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane

Parmi les dérivés du dibenzoylméthane mentionnés ci-dessus, on préfère tout particulièrement, selon la présente invention, mettre en oeuvre le 4-(ter.-butyl) 4'-méthoxy dibenzoylméthane, notamment celui proposé à la vente sous la dénomination commerciale de "PARSOL 1789" par la Société GIVAUDAN, ce filtre répondant donc à la formule développée suivante :

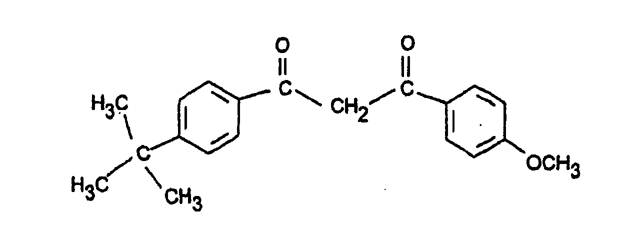

Un autre dérivé du dibenzoylméthane préféré selon la présente invention est le 4-isopropyl-dibenzoylméthane, filtre vendu sous la dénomination de "EUSOLEX 8020" par la Société MERCK, et répondant à la formule développée suivante :

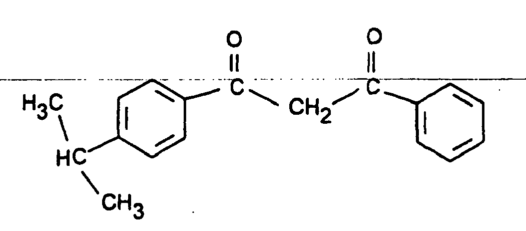

Le ou les dérivés du dibenzoylméthane peuvent être présents dans les compositions conformes à l'invention, ou dans les compositions destinées à être stabilisées conformément au procédé de l'invention, à des teneurs qui sont généralement comprises entre 0,01 % et 10 % en poids, et de préférence à des teneurs comprises entre 0,1 % et 6 % en poids, par rapport au poids total de la composition.

Les polymères épaississants conformes à la présente invention d'utilisation sont des copolymères comprenant au moins un motif hydrophobe tel que défini dans la revendication 1 dans une quantité suffisante pour obtenir sa solubilité partielle ou totale dans une phase huileuse et au moins un motif hydrophile dans une quantité suffisante pour produire un épaississement de ladite phase huileuse ; ledit motif hydrophile étant choisi parmi les monoacides carboxyliques à insaturation α,β-éthylénique en C₃-C₆, les diacides carboxyliques à insaturation α,β-éthylénique en C₄-C₆ ainsi que les dérivés monesters ou monoamides desdits diacides.

Ils sont choisis parmi ceux décrits et préparés dans le brevet US 5,736,125 (faisant partie intégrante du contenu de la demande).

Le ou les motifs hydrophobes desdits copolymères épaississants sont constitués par des (méth)acrylates d'alkyle en C₁₀-C₂₂ ; des (méth)acrylamides d'alkyle en C₁₀-C₂₂ ; les esters et éthers vinyliques en C₁₀-C₂₂ ; des siloxanes ; des α-oléfines en C₁₀-C₂₂ ; des chaînes aliphatiques latérales d'au moins 6 atomes de carbone fluorées ; des chaînes aliphatiques latérales d'alkyl(C₁-C₂₄)styrène d'au moins 6 atomes de carbone et plus particulièrement par les (méth)acrylates en C₁₈-C₂₂. Le ou les motifs hydrophobes représentent en général de 80 à 98% en poids et de préférence de 85 à 97% du poids total du copolymère épaississant.

Le ou les motifs hydrophiles desdits copolymères épaississants, nécessaire pour épaissir la phase huileuse peuvent être constitués par l'acide acrylique, l'acide (méth)acrylique ; l'acide maléique ou l'acide itaconique ou leurs monoesters ou monoamides d'alcools en C₁-C₂₂ et plus particulièrement par l'acide acrylique et/ou l'acide méthacrylique.

Les copolymères épaississants conformes à l'invention ont en général un indice d'acide allant de 0,1 à 4,0 meq/g et plus particulièrement de 0,4 à 2 meq/g. Leur poids moléculaire moyen est d'au moins 50.000 daltons et varie de préférence de 50.000 à 200.000 daltons.

Les copolymères épaississants de l'invention d'utilisation particulièrement préférés sont choisis parmi les copolymères (méth)acrylate d'alkyle en C₁₀-C₂₂/acide (méth)acrylique dans lesquels la quantité de (méth)acrylate d'alkyle est suffisante pour obtenir la solubilité partielle ou totale dudit polymère dans une phase huileuse et la quantité d'acide (méth)acrylique est suffisante pour épaissir la phase huileuse ;

Les copolymères épaississants conformément à l'invention d'utilisation susmentionnée sont encore plus particulièrement choisis parmi et les copolymères épaississants conformément à l'invention de la procédure susmentionnée et les inventions de la composition susmentionnée sont choisis parmi :
- les copolymères acrylate de docosyle/styrènelacide acrylique dans lesquels la quantité d'acrylate de docosyle et de styrène est suffisante pour obtenir une solubilité partielle ou totale dudit polymère dans une phase huileuse et la quantité d'acide acrylique est suffisante pour épaissir la phase huileuse tels que les produits décrits sous le noms SAMPLE 124-93 (72/4/2% en poids), SAMPLE 124-130 (68/27/5% en poids), SAMPLE 108-195 (67/28/5% en poids) dans le brevet US 5,736,125 et fabriqués par la Société LANDEC CORPORATION ;
- les copolymères acrylate de stéaryle/acide méthacrylique dans lesquels la quantité d'acrylate de stéaryle est suffisante pour obtenir une solubilité partielle ou totale dudit polymère dans une phase huileuse et la quantité d'acide méthacrylique est suffisante pour épaissir la phase huileuse ; tels que les produits décrits sous le noms SAMPLE 124-194 (92,5/7,5% en poids), SAMPLE 124-195 (90/10% en poids) dans le brevet US 5,736,125 et fabriqués par la Société LANDEC CORPORATION.

D'une manière générale, le ou les polymères épaississants peuvent ainsi être présents dans les compositions antisolaires selon l'invention, ou utilisés dans le procédé conforme à l'invention, à des teneurs qui sont généralement comprises entre 0,1 % et 10 % en poids, et de préférence à des teneurs comprises entre 0,2 % et 5 % en poids, par rapport au poids total de la composition.

Les compositions cosmétiques antisolaires photostables selon l'invention peuvent bien entendu contenir, outre les dérivés du dibenzoylméthane, un ou plusieurs filtres solaires complémentaires actifs dans l'UVA et/ou l'UVB, hydrophiles ou lipophiles. La présence de filtres complémentaires actifs dans l'UV-B (longueurs d'ondes comprise entre 280 nm et 320 nm environ) permet ainsi de disposer de compositions finales capables de filtrer l'ensemble des rayons UV.

Les compositions de l'invention peuvent aussi comprendre des adjuvants cosmétiques classiques notamment choisis parmi les solvants organiques, des épaississants ioniques ou non ioniques autres que ceux conformes à l'invention, les adoucissants, les antioxydants, les opacifiants, les stabilisants, les émollients, les silicones , les hydroxyacides, les agents anti-mousse, les agents hydratants, les vitamines, les parfums, les conservateurs, les tensioactifs, les charges, les pigments (minéraux ou organiques), les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions. Bien entendu, tous les ingrédients supplémentaires susceptibles d'être introduits dans les compositions conformes à l'invention doivent être tels qu'ils ne perturbent ou n'altèrent substantiellement pas l'effet de photostabilisation exercé par les copolymères épaississants sur les dérivés du dibenzoylméthane.

La phase huileuse des compositions photoprotectrices de l'invention peut être constituée par au moins une huile choisie parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées connues en soi. Elle peut contenir également des cires choisies parmi les cires animales, fossiles, végétales, minérales ou de synthèse connues en soi. Elle peut contenir également des acides gras, des alcools gras et des esters d'acides gras.

Afin d'obtenir une bonne photostabilité du dérivé de dibenzoylméthane, la phase huileuse des compositions photoprotectrices de l'invention ne contiendra pas de myristate d'isopropyle et/ou d'huile minérale lorsque le copolymère épaississant selon l'invention utilisé est le copolymère acrylate de stéaryle/acide méthacrylique (92,5/7,5% en poids).

Parmi les solvants organiques, on peut citer les alcools et polyols inférieurs.

Les épaississants additionnels peuvent être choisis notamment parmi les acides polyacryliques réticulés, les gommes de guar et celluloses modifiées ou non telles que la gomme de guar hydroxypropylée, la méthylhydroxy-éthylcellulose, l'hydroxypropylméthyl cellulose et l'hydroxyéthyl cellulose.

Les compositions de l'invention peuvent être préparées selon les techniques bien connues de l'homme de l'art, en particulier celles destinées à la préparation d'émulsions de type huile-dans-eau ou eau-dans-huile.

Ces compositions peuvent se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2315991 et FR 2416008).

Les compositions cosmétiques photostables de l'invention peuvent être utilisées comme compositions protectrices de l'épiderme humain ou des cheveux contre les rayons ultraviolets, comme compositions antisolaires ou encore comme produits de maquillage.

Lorsque les compositions cosmétiques selon l'invention sont utilisées pour la protection de l'épiderme humain contre les rayons UV, ou comme composition antisolaire, elles peuvent se présenter sous forme d'huile gélifiée, de suspension ou de dispersion dans des corps gras, sous forme de dispersion vésiculaire non ionique ou encore sous forme d'émulsion, de préférence de type huile-dans-eau, telle qu'une crème ou un lait, sous forme de pommade, de gel, de bâtonnet solide, de stick, de mousse aérosol ou de spray.

Lorsque les compositions cosmétiques selon l'invention sont utilisées pour la protection des cheveux, elles peuvent se présenter sous forme de shampooing, de lotion, de gel, d'émulsion, de dispersion vésiculaire non ionique, peut constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque les compositions sont utilisées comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elles peuvent se présenter sous forme solide ou pâteuse, anhydre ou aqueuse, comme des émulsions huile dans eau ou eau dans huile, des dispersions vésiculaires non ioniques ou encore des suspensions.

Des exemples concrets, mais nullement limitatifs, illustrant l'invention, vont maintenant être donnés.

### EXEMPLE 1

Dans cet exemple, on a étudié la photostabilité du 4-(ter.-butyl) 4'-méthoxy dibenzoylméthane (filtre solaire "PARSOL 1789" de chez GIVAUDAN) en présence d'un copolymère épaississant selon l'invention à savoir : copolymères acrylate de stéaryle/acide méthacrylique (92,5/7,5% en poids) décrit sous le nom SAMPLE 124-194 dans le brevet US 5,736,125.

A titre comparatif, on a étudié la photostabilité de ce même filtre en l'absence de ce polymère épaississant.

On a également étudié la photostabilité du 4-(ter.-butyl)-4'-méthoxy dibenzoylméthane en faisant varier la quantité de polymère épaississant.

Les compositions de ces trois formules (F0-F1-F2) étaient ainsi les suivantes (% en poids par rapport au poids total de la formule) :

| | | |
|---|---|---|
| F0 (comparatif) | Polymère épaississant 0 % | Support commun^{*} |
| F1 (invention) | Polymère épaississant 1,9 % | Support commun^{*} |
| F2 (invention) | Polymère épaississant 3 % | Support commun^{*} |

| | | |
|---|---|---|
| ^{*}la composition du support commun était elle-même la suivante (% en poids par rapport au poids total de la formule) : - Polymère épaississant SAMPLE 124-194 de chez LANDEC CORPX % - 4-tert.butyl-4'-méthoxydibenzoylméthane vendu sous le nom   PARSOL 1789 par la société GIVAUDAN 2% - Benzoates d'alkyle en C₁₂/C₁₅ vendu sous le nom   FINSOLV TN par la société FINETEX 4% - Hexyldécanol/hexyldecyl-laurate vendu sous le nom   CETIOL PLG de la société Henkel. qsp 100 % | | |

La photostabilité du filtre solaire dans ces formulations a été quantifiée par dosage spectrophotométrique du filtre résiduel après deux heures d'irradiation au moyen d'un simulateur solaire. Le protocole opératoire exact qui a été suivi est le suivant :
- les formules préparées sont étalées à raison de 2 mg/cm² sur un support en polyméthylméthacrylate dépoli ;
- les échantillons sont ensuite soumis pendant deux heures, à température constante, au rayonnement d'un Suntest HERAEUS (Source : Arc long Xenon 1,8 kW), afin de simuler une irradiation UV naturelle (UV-A + UV-B) ;
- après exposition, chaque échantillon est plongé dans 55 ml de méthanol pour extraction du filtre solaire ;
- les compositions ainsi obtenues sont analysées par spectrophotométrie UV dans la plage 290-400 nm.

Le taux de filtre résiduel après irradiation s'exprime mathématiquement par le rapport entre la concentration en filtre mesurée dans l'échantillon irradié et la concentration initiale de ce filtre dans l'échantillon avant irradiation.

Les résultats obtenus ont été les suivants :

| **Compositions** | **PARSOL 1789 résiduel après 2 heures d'irradiation** |
|---|---|
| F0 (comparatif) | 4,07 % (± 3,75) |
| F1 (invention) | 35,74 % (± 5,55) |
| F2 (invention) | 43,20 % (± 4,65) |

Ces résultats mettent clairement en évidence l'effet de photostabilisation remarquable apporté par le polymère épaississant conforme à l'invention sur le 4-(ter.-butyl) 4'-méthoxy dibenzoylméthane.

## Revendications

1. Utilisation d'un copolymère épaississant pour stabiliser vis-à-vis du rayonnement UV un dérivé du dibenzoylméthane contenu dans une composition cosmétique comprenant au moins une phase huileuse, le copolymère épaississant comprenant
(i) au moins un motif hydrophobe choisi parmi
a) les (méth)acrylates d'alkyle en C₁₀-C₂₂;
b) les (méth)acrylamides d'alkyle en C₁₀-C₂₂;
c) les esters et éthers vinyliques en C₁₀-C₂₂;
d) des siloxanes;
e) des α-oléfines en C₁₀-C₂₂;
f) les chaînes aliphatiques latérales d'au moins 6 atomes de carbone fluorées
h) ou les chaînes aliphatiques latérales d'alkyl(C₁-C₂₄)styrène d'au moins 6 atomes de carbone,
dans une quantité suffisante pour obtenir sa solubilité partielle ou totale dans ladite phase huileuse et
(ii) au moins un motif hydrophile choisi parmi
a) les monoacides carboxyliques à insaturation α,β-éthylénique en C₃-C₆,
b) les diacides carboxyliques à insaturation α,β-éthylénique en C₄-C₆,
c) ainsi que les dérivés monoesters ou monoamides desdits diacides,
dans une quantité suffisante pour produire un épaississement de ladite phase huileuse.

2. Utilisation selon la revendication 1, **caractérisé par le fait que** le ou les motifs hydrophobes dudit copolymère épaississant est constitué par des (méth)acrylates d'alkyle en C₁₈-C₂₂.

3. Utilisation selon l'une quelconque des revendications 1 ou 2, **caractérisé par le fait que** le ou les motifs hydrophobes dudit copolymère épaississant représentent de 80 à 98% et de préférence de 85 à 97% du poids total du copolymère épaississant.

4. Utilisation selon l'une quelconque des revendications 1 à 3, **caractérisé par le fait que** le ou les motifs hydrophiles dudit copolymère épaississant sont constitués par l'acide acrylique ou l'acide (méth)acrylique; l'acide maléique ou l'acide itaconique ou leurs monoesters ou monoamides d'alcools en C₁-C₂₂.

5. Utilisation selon l'une quelconque des revendications 1 à 4, **caractérisé par le fait que** le copolymère épaississant présente un indice d'acide allant de 0,1 à 4,0 meq/g et plus particulièrement de 0,4 à 2 meq/g.

6. Utilisation selon l'une quelconque des revendications 1 à 5, **caractérisé par le fait que** le copolymère épaississant présente un poids moléculaire d'au moins 50.000 daltons et varie de préférence de 50.000 à 200.000 daltons.

7. Utilisation selon l'une quelconque des revendications 1 à 6, **caractérisé par le fait que** le copolymère épaississant est choisi parmi les copolymères (méth)acrylate d'alkyle en C₁₀-C₂₂/acide (méth)acrylique dans lesquels la quantité de (méth)acrylate d'alkyle est suffisante pour produire une solubilité partielle ou totale dudit polymère dans une phase huileuse et la quantité d'acide (méth)acrylique est suffisante pour épaissir la phase huileuse.

8. Utilisation selon la revendication 7, où le copolymère épaississant est choisi parmi les copolymères acrylate de docosyle/styrène/acide acrylique et les copolymères acrylate de stéaryle/acide méthacrylique dans lesquels la quantité de d'acrylate de docosyle et de styrène est suffisante pour produire une solubilité partielle ou totale dudit polymère dans une phase huileuse et la quantité d'acide acrylique est suffisante pour épaissir la phase huileuse ou dans lesquels la quantité d'acrylate de stéaryle est suffisante pour obtenir une solubilité partielle ou totale dudit polymère dans une phase huileuse et la quantité d'acide méthacrylique est suffisante pour épaissir la phase huileuse.

9. Utilisation selon la revendication 8, où le copolymère épaississant est choisis parmi
- le copolymère acrylate de docosyle/styrène/acide acrylique (72/4/2% en poids)
- le copolymère acrylate de docosyle/styrène/acide acrylique (68/27/5% en poids)
- le copolymère acrylate de docosyle/styrène/acide acrylique (67/28/5% en poids)
- le copolymère acrylate de stéaryle/acide méthacrylique (92,5/7,5% en poids)
- le copolymère acrylate de stéaryle/acide méthacrylique (90/10% en poids).

10. Utilisation selon l'une quelconque des revendications 1 à 9, où ladite phase huileuse ne contient pas de myristate d'isopropyle et/ou d'huile minérale lorsque le copolymère épaississant est le copolymère acrylate de stéaryle/acide méthacrylique (92,5/7,5% en poids).

11. Procédé de stabilisation d'au moins un dérivé du dibenzoylméthane vis-à-vis du rayonnement ultraviolet dans un support comprenant au moins une phase huileuse, **caractérisé par le fait qu'**il consiste à associer audit dérivé du dibenzoylméthane une quantité efficace d'au moins un copolymère épaississant tel que défini dans la revendication 8, sous réserve que ladite phase huileuse ne contient pas de myristate d'isopropyle et/ou d'huile minérale lorsque le copolymère épaississant est le copolymère acrylate de stéaryle/acide méthacrylique 92,5/7,5% en poids).

12. Procédé selon la revendication 11, où le ou les dérivés du dibenzoylméthane sont choisis dans le groupe constitué par:
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4,4'-diméthoxydibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane

13. Procédé selon la revendication 12, où le dérivé du dibenzoylméthane est le 4-(ter.-butyl) 4'-méthoxy dibenzoylméthane.

14. Procédé selon la revendication 12, où le dérivé du dibenzoylméthane est le 4-isopropyl-dibenzoylméthane.

15. Composition cosmétique filtrante photostable pour la photoprotection par voie topique de la peau et/ou des cheveux contre le rayonnement ultraviolet, en particulier le rayonnement solaire, du type comprenant, dans un support cosmétiquement acceptable, au moins un dérivé du dibenzoylméthane et au moins une phase huileuse, **caractérisée par le fait qu'**elle comprend en plus une quantité efficace d'au moins un copolymère épaississant tel que défini dans la revendication 8, sous réserve que ladite phase huileuse ne contient pas de myristate d'isopropyle et/ou d'huile minérale lorsque le copolymère épaississant est le copolymère acrylate de stéaryle/acide méthacrylique (92,5/7,5% en poids).

16. Composition selon la revendication 15, **caractérisées par le fait que** la teneur en dérivé(s) du dibenzoylméthane est comprise entre 0,01 % et 10 % en poids par rapport au poids total de la composition.

17. Composition selon la revendication 16, **caractérisée par le fait que** ladite teneur est comprise entre 0,1 % et 6 % en poids par rapport au poids total de la composition.

18. Composition selon l'une quelconque des revendications 15 à 17, **caractérisée par le fait que** la teneur en copolymère(s) épaississant varie de 0,1% à 10 % en poids par rapport au poids total de la composition.

19. Composition selon la revendication 18, **caractérisée par le fait que** ladite teneur varie de 0,2% à 5 % en poids par rapport au poids total de la composition.

## Patentansprüche

1. Verwendung eines verdickenden Copolymers zur Stabilisierung eines Dibenzoylmethanderivats gegenüber UV-Strahlung, das in einer kosmetischen Zusammensetzung vorhanden ist, umfassend mindestens eine Ölphase, wobei das verdickende Copolymer umfasst:
(I) mindestens eine hydrophobe Einheit, ausgewählt aus
a) C₁₀-C₂₂-Alkyl(meth)acrylaten;
b) C₁₀-C₂-Alkyl(meth)acrylamiden;
c) C₁₀-C₂₂-Vinylestern und -ethem;
d) Siloxanen;
e) C₁₀-C₂₂-α-Olefinen;
f) aliphatischen Seitenketten mit mindestens 6 fluorierten Kohlenstoffatomen;
h) oder aliphatischen Seitenketten von (C₁-C₂₄)-Alkylstyrol mit mindestens 6 Kohlenstoffatomen,
in einer Menge, die ausreichend ist, damit es in der Ölphase teilweise oder ganz löslich ist, und
(II) mindestens eine hydrophile Einheit, ausgewählt aus
a) α,β-ethylenisch ungesättigten C₃-C₆-Monocarbonsäuren,
b) α,β-ethylenisch ungesättigten C₄-C₆-Dicarbonsäuren,
c) und den Monoester- oder Monoamidderivaten der Disäuren,
in einer Menge, die ausreichend ist, um eine Verdickung der Ölphase zu erreichen.

2. Verwendung nach Anspruch 1, **dadurch gekennzeichnet, dass** die hydrophobe(n) Einheit(en) des verdickenden Copolymers aus C₁₈-C₂₂-Alkyl(meth)acrylaten bestehen.

3. Verwendung nach einem der Ansprüche 1 oder 2, **dadurch gekennzeichnet, dass** die hydrophobe(n) Einheit(en) des verdickenden Copolymers 80 bis 98 % und vorzugsweise 85 bis 97 % des Gesamtgewichts des verdickenden Copolymers ausmachen.

4. Verwendung nach einem der Ansprüche 1 bis 3, **dadurch gekennzeichnet, dass** die hydrophile(n) Einheit(en) des verdickenden Copolymers aus Acrylsäure oder Methacrylsäure; Maleinsäure oder Itaconsäure oder deren Monoestem oder Monoamiden, die von C₁-C₂₂-Alkoholen abgeleitet sind, bestehen.

5. Verwendung nach einem der Ansprüche 1 bis 4, **dadurch gekennzeichnet, dass** das verdickende Copolymer eine Säurezahl im Bereich von 0,1 bis 4,0 meq/g und insbesondere 0,4 bis 2 meq/g aufweist.

6. Verwendung nach einem der Ansprüche 1 bis 5, **dadurch gekennzeichnet, dass** das verdickende Copolymer ein Molekulargewicht von mindestens 50.000 Dalton und vorzugsweise im Bereich von 50.000 bis 200.000 Dalton aufweist.

7. Verwendung nach einem der Ansprüche 1 bis 6, **dadurch gekennzeichnet, dass** das verdickende Copolymer aus C₁₀-C₂₂-Alkyl(meth)acrylat/(Meth)acrylsäure-Copolymeren ausgewählt ist, bei denen die Menge an Alkyl(meth)acrylat ausreichend ist, damit das Polymer in einer Ölphase teilweise oder ganz löslich ist, und die Menge an (Meth)acrylsäure ausreichend ist, um die Ölphase einzudicken.

8. Verwendung nach Anspruch 7, wobei das verdickende Copolymer aus Docosylacrylat/Styrol/Acrylsäure-Copolymeren und Stearylacrylat/Methacrylsäure-Copolymeren ausgewählt ist, bei denen die Menge an Docosylacrylat und Styrol ausreichend ist, damit das Polymer in einer Ölphase teilweise oder ganz löslich ist, und die Menge an Acrylsäure ausreichend ist, um die Ölphase einzudicken, oder bei denen die Menge an Stearylacrylat ausreichend ist, damit das Polymer in einer Ölphase teilweise oder ganz löslich ist, und die Menge an Methacrylsäure ausreichend ist, um die Ölphase einzudicken.

9. Verwendung nach Anspruch 8, wobei das verdickende Copolymer ausgewählt ist aus
- Docosylacrylat/Styrol/Acrylsäure-Copolymer (72/4/2 Gew.-%)
- Docosylacrylat/Styrol/Acrylsäure-Copolymer (68/27/5 Gew.-%)
- Docosylacrylat/Styrol/Acrylsäure-Copolymer (67/28/5 Gew.-%)
- Stearylacrylat/Methacrylsäure-Copolymer (92,5/7,5 Gew.-%)
- Stearylacrylat/Methacrylsäure-Copolymer (90/10 Gew.-%).

10. Verwendung nach einem der Ansprüche 1 bis 9, wobei die Ölphase kein Isopropylmyristat und/oder Mineralöl enthält, wenn das verdickende Copolymer das Stearylacrylat/Methacrylsäure-Copolymer (92,5/7,5 Gew.-%) ist.

11. Verfahren zur Stabilisierung mindestens eines Dibenzoylmethanderivats gegenüber Ultraviolett-Strahlung in einem Träger, der mindestens eine Ölphase enthält, **dadurch gekennzeichnet, dass** es darin besteht, mit dem Dibenzoylmethanderivat mindestens ein verdickendes Copolymer wie in Anspruch 8 definiert in einer wirksamen Menge zu kombinieren, mit der Maßgabe, dass die Ölphase kein Isopropylmyristat und/oder Mineralöl enthält, wenn das verdickende Copolymer das Stearylacrylat/Methacrylsäure-Copolymer (92,5/7,5 Gew.-%) ist.

12. Verfahren nach Anspruch 11, wobei das Dibenzoylmethanderivat oder die Dibenzoylmethanderivate aus der Gruppe ausgewählt sind, die besteht aus:
- 2-Methyldibenzoylmethan
- 4-Methyldibenzoylmethan
- 4-Isopropyldibenzoylmethan
- 4-tert-Butyldibenzoylmethan
- 2,4-Dimethyldibenzoylmethan
- 2,5-Dimethyldibenzoylmethan
- 4,4'-Diisopropyldibenzoylmethan
- 4,4'-Dimethoxydibenzoylmethan
- 4-tert-Butyl-4'-methoxydibenzoylmethan
- 2-Methyl-5-isopropyl-4'-methoxydibenzoylmethan
- 2-Methyl-5-tert-butyl-4'-methoxydibenzoylmethan
- 2,4-Dimethyl-4'-methoxydibenzoylmethan
- 2,6-Dimethyl-4-tert-butyl-4'-methoxydibenzoylmethan.

13. Verfahren nach Anspruch 12, wobei das Dibenzoylmethanderivat 4-(tert-Butyl)-4'-methoxydibenzoylmethan ist.

14. Verfahren nach Anspruch 12, wobei das Dibenzoylmethanderivat 4-Isopropyldibenzoylmethan ist.

15. Photostabile filternde kosmetische Zusammensetzung für den Lichtschutz der Haut und/oder Haare gegenüber Ultraviolett-Strahlung, insbesondere Sonnenstrahlung, auf topischem Wege des Typs, der in einem kosmetisch akzeptablen Träger mindestens ein Dibenzoylmethanderivat und mindestens eine Ölphase enthält, **dadurch gekennzeichnet, dass** sie ferner eine wirksame Menge mindestens eines verdickenden Copolymers wie in Anspruch 1 definiert enthält, mit der Maßgabe, dass die Ölphase kein Isopropylmyristat und/oder Mineralöl enthält, wenn das verdickende Copolymer das Stearylacrylat/Methacrylsäure-Copolymer (92,5/7,5 Gew.-%) ist.

16. Zusammensetzung nach Anspruch 15, **dadurch gekennzeichnet, dass** der Mengenanteil an Dibenzoylmethanderivat(en) zwischen 0,01 Gew.-% und 10 Gew.-%, bezogen auf das Gesamtgewicht der Zusammensetzung, liegt.

17. Zusammensetzung nach Anspruch 16, **dadurch gekennzeichnet, dass** der Mengenanteil zwischen 0,1 Gew.-% und 6 Gew.-%, bezogen auf das Gesamtgewicht der Zusammensetzung, liegt.

18. Zusammensetzung nach einem der Ansprüche 15 bis 17, **dadurch gekennzeichnet, dass** der Mengenanteil an verdickendem (verdickenden) Copolymer(en) im Bereich von 0,1 Gew.-% bis 10 Gew.-%, bezogen auf das Gesamtgewicht der Zusammensetzung, liegt.

19. Zusammensetzung nach Anspruch 18, **dadurch gekennzeichnet, dass** der Mengenanteil im Bereich von 0,2 Gew.-% bis 5 Gew.-%, bezogen auf das Gesamtgewicht der Zusammensetzung, liegt.

## Claims

1. Use of a thickening copolymer for stabilizing, with respect to UV radiation, a dibenzoylmethane derivative present in a cosmetic composition comprising at least one oily phase, the thickening copolymer comprising:
(I) at least one hydrophobic unit chosen from
a) C₁₀-C₂₂ alkyl (meth)acrylates;
b) C₁₀-C₂₂ alkyl (meth)acrylamides;
c) C₁₀-C₂₂ vinyl esters and ethers;
d) siloxanes;
e) C₁₀-C₂₂ α-olefins;
f) aliphatic side chains with at least 6 fluorinated carbon atoms;
h) or aliphatic side chains of (C₁-C₂₄) alkylstyrene with at least 6 carbon atoms,
in an amount sufficient to result in its partial or complete solubility in said oily phase, and
(II) at least one hydrophilic unit chosen from
a) α, β-ethylenically unsaturated C₃-C₆ monocarboxylic acids,
b) α, β-ethylenically unsaturated C₄-C₆ dicarboxylic acids,
c) and the monoester or monoamide derivatives of said diacids,
in an amount sufficient to produce thickening of said oily phase.

2. Use according to claim 1, **characterized in that** the hydrophobic unit or units of said thickening copolymer are composed of C₁₈-C₂₂ alkyl (meth)acrylates.

3. Use according to any one of claims 1 or 2, **characterized in that** the hydrophobic unit or units of said thickening copolymer represent from 80 to 98 % and preferably from 85 to 97 % of the total weight of the thickening copolymer.

4. Use according to any one of claims 1 to 3, **characterized in that** the hydrophilic unit or units of said thickening copolymer are composed of acrylic acid or methacrylic acid; maleic acid or itaconic acid or their monoesters or monoamides derived from C₁-C₂₂ alcohols.

5. Use according to any one of claims 1 to 4, **characterized in that** the thickening copolymer exhibits an acid number ranging from 0.1 to 4.0 meq/g and more particularly from 0.4 to 2 meq/g.

6. Use according to any one of claims 1 to 5, **characterized in that** the thickening copolymer exhibits a molecular weight of at least 50.000 daltons and preferably varying from 50.000 to 200.000 daltons.

7. Use according to any one of claims 1 to 6, **characterized in that** the thickening copolymer is chosen from C₁₀-C₂₂ alkyl (meth)acrylate/(meth)acrylic acid copolymers in which the amount of alkyl (meth)acrylate is sufficient to result in a partial or complete solubility of said polymer in an oily phase and the amount of (meth)acrylic acid is sufficient to thicken the oily phase.

8. Use according to claim 7, wherein the thickening copolymer is chosen from docosyl acrylate/styrene/acrylic acid copolymers and stearyl acrylate/methacrylic acid copolymers in which the amount of docosyl acrylate and of styrene is sufficient to result in a partial or complete solubility of said polymer in an oily phase and the amount of acrylic acid is sufficient to thicken the oily phase or in which the amount of stearyl acrylate is sufficient to result in a partial or complete solubility of said polymer in an oily phase and the amount of methacrylic acid is sufficient to thicken the oily phase.

9. Use according to claim 8, wherein the thickening copolymer is chosen from
- docosyl acrylate/styrene/acrylic acid (72/4/2 % by weight) copolymer
- docosyl acrylate/styrene/acrylic acid (68/27/5 % by weight) copolymer
- docosyl acrylate/styrene/acrylic acid (67/28/5 % by weight) copolymer
- stearyl acrylate/methacrylic acid (92.5/7.5 % by weight) copolymer
- stearyl acrylate/methacrylic acid (90/10 % by weight) copolymer.

10. Use according to any one of claims 1 to 9, wherein said oily phase does not comprise isopropyl myristate and/or mineral oil, when the thickening copolymer is the stearyl acrylate/methacrylic acid (92.5/7.5 % by weight) copolymer.

11. Process for the stabilization, with respect to ultraviolet radiation, of at least one dibenzoylmethane derivative in a vehicle comprising at least one oily phase, **characterized in that** it consists of combining said dibenzoylmethane derivative with an effective amount of at least one thickening copolymer as defined in claim 8, with the proviso that said oily phase does not comprise isopropyl myristate and/or mineral oil, when the thickening copolymer is the stearyl acrylate/methacrylic acid (92.5/7.5 % by weight) copolymer.

12. Process according to claim 11, wherein the dibenzoylmethane derivative or derivatives are chosen from the group composed of:
- 2-methyldibenzoylmethane
- 4-methyldibenzoylmethane
- 4-isopropyldibenzoylmethane
- 4-tert-butyldibenzoylmethane
- 2,4-dimethyldibenzoylmethane
- 2,5-dimethyldibenzoylmethane
- 4,4'-diisopropyldibenzoylmethane
- 4,4'-dimethoxydibenzoylmethane
- 4-tert-butyl-4'-methoxydibenzoylmethane
- 2-methyl-5-isopropyl-4'-methoxydibenzoylmethane
- 2-methyl-5-tert-butyl-4'-methoxydibenzoylmethane
- 2,4-dimethyl-4'-methoxydibenzoylmethane
- 2,6-dimethyl-4-tert-butyl-4'-methoxydibenzoylmethane.

13. Process according to claim 12, wherein the dibenzoylmethane derivative is 4-(tert-butyl)-4'-methoxydibenzoylmethane.

14. Process according to claim 12, wherein the dibenzoylmethane derivative is 4-isopropyldibenzoylmethane.

15. Photostable screening cosmetic composition for the photoprotection, by the topical route, of the skin and/or hair against ultraviolet radiation, in particular solar radiation, of the type comprising, in a cosmetically acceptable vehicle, at least one dibenzoylmethane derivate and at least one oily phase, **characterized in that** it additionally comprises an effective amount of at least one thickening copolymer as defined in claim 1, with the proviso that said oily phase does not comprise isopropyl myristate and/or mineral oil, when the thickening copolymer is the stearyl acrylate/methacrylic acid (92.5/7.5 % by weight) copolymer.

16. Composition according to claim 15, **characterized in that** the content of dibenzoylmethane derivative(s) is between 0.01 % and 10 % by weight with respect to the total weight of the composition.

17. Composition according to claim 16, **characterized in that** said content is between 0.1 % and 6 % by weight with respect to the total weight of the composition.

18. Composition according to any one of claims 15 to 17, **characterized in that** the content of thickening copolymer(s) varies from 0.1 % to 10 % by weight with respect to the total weight of the composition.

19. Composition according to claim 18, **characterized in that** said content varies from 0.2 % to 5 % by weight with respect to the total weight of the composition.
